# EUROPEAN PATENT APPLICATION

(11) **EP 1 604 613 A1**
(43) Date of publication of application: **14.12.2005**
(21) Application number: 04711741.1
(22) Date of filing: 17.02.2004
(51) Int. Cl.: A61B 8/12, A61B 5/00, G01N 3/40

(54) **CATHETER SENSOR FOR MEASURING HARDNESS**

(30) Priority: 20.02.2003 JP 2003042127
(71) Applicant: Nihon University, Tokyo 102-8275 (JP)
(72) Inventor: Omata, Sadao, c/o Nihon University, Tokyo 1028275 (JP)
(74) Representative: Hallybone, Huw George
(86) International application number: PCT/JP2004/001740
(87) International publication number: WO 2004/073522

(57) **Abstract**

A catheter sensor fabricated by applying a hardness sensor having a simple structure and being advantageous for the miniaturization to a catheter is provided. The catheter sensor for measuring hardness of a portion to be measured in a blood vessel or the other organ comprises a casing (13), that contains an oscillation section (11) for emitting oscillation to a portion to be measured and an oscillating wave reception section (12) for receiving reflective oscillation, and a signal processing section (20) for calculating hardness of a portion to be measured on the basis of a change in the phases of the emitted oscillation of the oscillation section and the reflective oscillation of the oscillating wave reception section. The casing is filled with a kind of liquid (14). The emitted oscillation from the oscillation section is transmitted to the portion to be measured through the liquid, and the reflective oscillation reflected from the portion to be measured is transmitted to the oscillating wave reception section through the liquid.

## Description

### Field of the Invention

The present invention relates to a catheter sensor to be used for an objects being in a thinly-tubular form and particularly to a catheter sensor for measuring hardness of a portion to be measured, such as an inner wall of a blood vessel.

### Description of the Related Art

Until today, there have been various sensors for measuring hardness. There exist various needs to measure the hardness of objects, and in the medical field, there is not only a demand to display a state inside a blood vessel and the other organs in the form of an image by means of a fiber scope or the like but also a demand to measure the hardness of a lump and/or an agglomerate in a blood vessel and/or the other organs. Because, if the hardness of a lump in a blood vessel is known, it makes possible to more definitely understand, for example, what type of a lump is it, like knowing by conducting palpation.

A sensor that is a hardness sensor for measuring hardness and uses a vibrator to measure hardness based on a change in the phases of emitted oscillation and reflective oscillation is disclosed in JP Patent Laid-open No. H09-145691. Although this sensor may be inserted in a body to measure a hardness of an organ, the sensor cannot be downsized to such an extent that it can measure a hardness of a blood vessel and the like since the sensor section thereof is formed in a complex structure.

A measuring apparatus that measures a hardness of an object to be measured with no contact with the object is disclosed in WO 01/84135. Since this measuring apparatus is mainly directed to signal processing, no disclosure is made on the specific structure of the sensor unit included in the measuring apparatus. Further, there is no description about the structure, etc. to be applied for a sensor in a compact size such as a catheter that is used for measuring hardness inside a blood vessel and the like.

However, since the conventional hardness sensors do not suit to be structured in a compact size as described above, it has been difficult to insert the sensor into a blood vessel and to then measure the hardness of a lump and/or an agglomerate existing in the inner wall of the blood vessel. In particular, even though it is intended to apply a hardness sensor for a thin catheter having a diameter of 1 mm more or less, it is difficult to make the sensor into a fine size if the vibrator included therein has a complex structure. Accordingly, it has been difficult to make the diameter of a catheter thinner containing a hardness sensor. Therefore, a sensor structured in a fine size and being capable of measuring the hardness inside a blood vessel has been desired.

Taking the above-described difficulty into consideration, it is an object of the present invention to provide a catheter sensor for hardness measurement purpose, which can build a hardness sensor being simply structured and advantageously downsized in a catheter.

### SUMMARY OF THE INVENTION

In order to achieve the object of the present invention as described above, the present invention provides a catheter sensor for hardness measurement use comprising an oscillation section that oscillates at a prefixed frequency and emits oscillation to a portion to be measured, an oscillating wave reception section for receiving reflective oscillation which is reflected from the portion to be measured, against which the emitted oscillation from the oscillation section is emitted, a casing containing the oscillation section and the oscillating wave reception section, a signal processing section for calculating the hardness of the portion to be measured on the basis of a change in the phases of the emitted oscillation of the oscillation section and the reflective oscillation of the oscillating wave reception section, and a kind of liquid filled in the casing. The emitted oscillation from the oscillation section is transmitted to the portion to be measured through the liquid, and the reflective oscillation from the portion to be measured is transmitted to the oscillating wave reception section through the liquid.

In the catheter sensor described above, an expandable balloon section is provided in the casing, which is adapted to expand to contact with the portion to be measured in response to the pressure elevation in the liquid filled in the casing. The oscillation emitted from the oscillation section may be transmitted by passing through the liquid to reach the portion to be measured via the balloon section.

Besides, the oscillation section and the oscillating wave reception section may also be provided on a portion that locates at the end side of the catheter sensor being opposite to the side of the portion to be measured, that is a portion to be inserted into a blood vessel or the like, and is not inserted into a blood vessel or the like.

Alternatively, the oscillation section may be configured such that it emits oscillation toward the longitudinal direction of the catheter sensor, the catheter sensor may be provided with a reflection section that reflects the oscillation emitted from the oscillation section to a direction perpendicular to the longitudinal direction of the catheter sensor, and the reflection section may be arranged in the casing. The reflection section, instead thereof, may be rotatably arranged around the longitudinal direction of the catheter sensor as an axis.

Alternatively, the oscillation section may also be configured such that it emits oscillation to a direction perpendicular to the longitudinal direction of the catheter sensor. In this case, the oscillation section may be rotatably arranged around the longitudinal direction of the catheter sensor as an axis.

Besides, the casing may be configured such that it can be rotatably provided around the longitudinal direction of the catheter sensor as an axis.

Further, the oscillation section and the oscillating wave reception section may comprise a single vibrator that may be provided with an input terminal for the oscillation section and an output terminal for the oscillating wave reception section, and the input and output terminals may be composed of a split electrode.

Alternatively, it may be configured such that the oscillation section and the oscillating wave reception section are composed of two vibrators, one of those which is provided with an input terminal for the oscillation section and a ground terminal, and the other of those which is provided with an output terminal for the oscillating wave reception section and a ground terminal.

In this context, the oscillation section and the oscillating wave reception section may be composed of any of a piezoelectric ceramic vibrator, a laminated piezoelectric ceramic vibrator, a bimorph vibrator, a crystal vibrator, a PVDF vibrator, a magnetostrictive element and an SAW, respectively. Additionally, the shapes of the oscillation section and the oscillating wave reception section may be cylindrical, columnar, prismatic or the like, respectively.

With the catheter sensor having the configuration as described above, the following advantageous effects can be attained. Because the catheter sensor for hardness measurement use according to the present invention is constructed in a simple structure, it can be downsized advantageously and is applicable for an extremely fine catheter sensor. Hence, with the catheter sensor of the present invention, a measurement of hardness of fine inner walls of blood vessels can be realized. In addition, the reflective plate and the sensor itself can be rotated to carry out the directional measurements so that information on the hardness of the portions to be measured in blood vessels can be displayed in visible manner.

### BRIEF DESCRIPTION OF THE DRAWINGS

FIG. 1 is a view for schematically explaining a catheter sensor for hardness measurement use according to the present invention,
FIG. 2 is a view for explaining a configuration of a signal processing section of a catheter sensor for hardness measurement use according to the present invention,
FIG. 3 is a view for explaining a configuration of a sensor section of a catheter sensor for hardness measurement use according to the present invention,
FIG. 4 is a view for explaining the other configuration of a sensor section of a catheter sensor for hardness measurement use according to the present invention,
FIG. 5 is a view for explaining the other configuration of a sensor section of a catheter sensor for hardness measurement use according to the present invention,
FIG. 6 is a view for explaining the rotational manner of a reflective plate attached in a sensor section of a catheter sensor for hardness measurement use according to the present invention,
FIG. 7 is a view depicting an exemplary display of a measurement result when hardness in a blood vessel is measured by means of a catheter sensor for hardness measurement use having a property of directionality,
FIG. 8 is a view for explaining the rotational manner of a sensor section of a catheter sensor for hardness measurement use according to the present invention,
FIG. 9 is a view for explaining a configuration with which a casing of a catheter sensor for hardness measurement use according to the present invention rotates,
FIG. 10 is a view for explaining a catheter sensor having a configuration in which a sensor section and a part reaching a portion to be measured of a catheter sensor for hardness measurement use according to the present invention are separated in a great distance and the sensor section is arranged in an area outside a blood vessel,
FIG. 11 is a view for explaining a structure of a vibrator to be used for a catheter sensor for hardness measurement use according to the present invention,
FIG. 12 is a view for explaining the shape of a vibrator to be used for a catheter sensor for hardness measurement use according to the present invention,
FIG. 13 is a graph showing a frequency-gain-phase characteristic curve that indicates an overall frequency characteristic obtained by conflating the respective frequency characteristics of a self-oscillation circuit and a phase-shifting circuit section, and
FIG. 14 is a graph showing a frequency-gain-phase characteristic curve that indicates the respective frequency characteristics of a self-oscillation circuit and a phase-shifting circuit section.

### DETAILED DESCRIPTION OF THE PREFERRED EMBODIMENTS

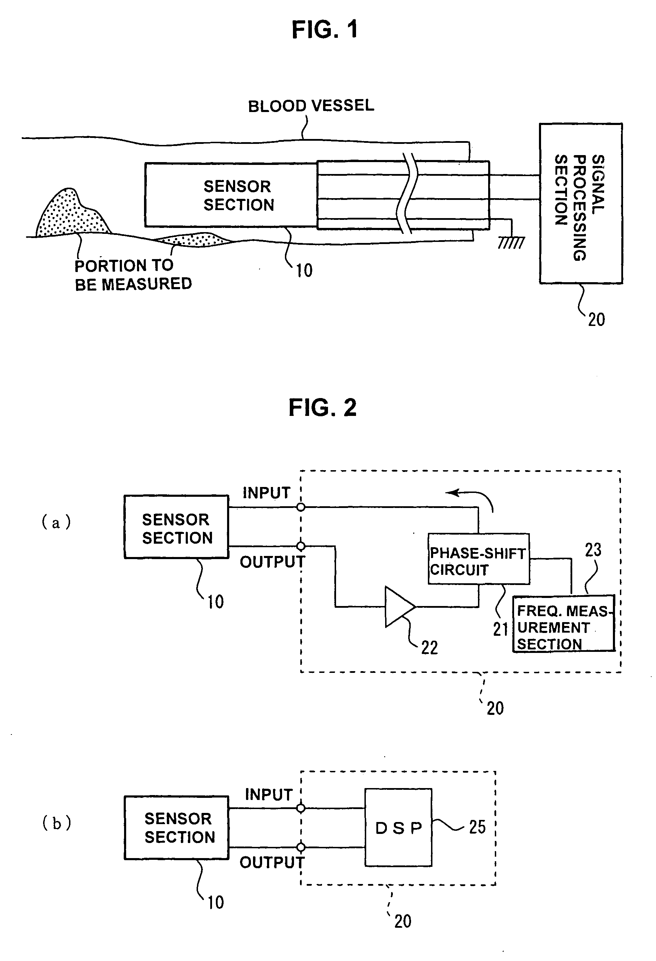

Now, the embodiments of the present invention including the examples shown in the appended drawings are described in the following. FIG. 1 is a schematic view of the catheter sensor for hardness measurement use according to the present invention, and a sensor section 10 in the state of being inserted into a blood vessel is shown in this drawing. With this catheter sensor, an oscillation such as ultrasonic waves is emitted to a portion to be measured, that is an affected part such as a tumor, the reflective oscillation reflected from the portion to be measured is received in the sensor section 10, and the hardness of the portion to be measured is determined in the signal processing section 20 based on a change in the phases of the emitted oscillation and the reflective oscillation.

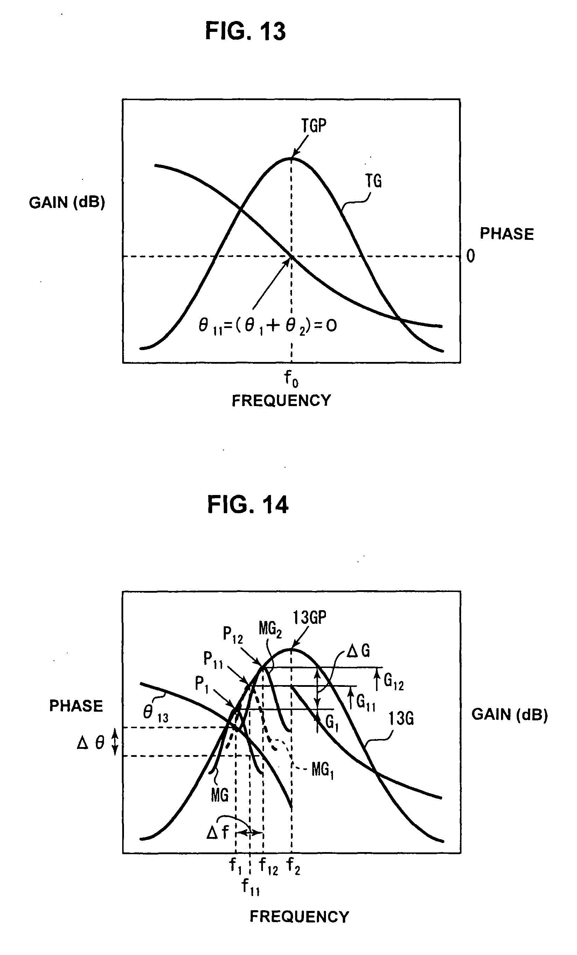

As shown in FIG. 2(a), in the signal processing section 20, a phase-shifting circuit section 21 and an amplifier circuit section 22 are provided between the input and output terminals of the sensor section 10 so that a self-oscillation circuit that makes the signal processing section to function as a compulsory feedback loop is formed, an output from the phase-shifting circuit section 21 is measured in a frequency measurement section 23, and an amount of change in the phases of the emitted oscillation and the reflective oscillation is measured based on the amount of change in the frequency so that a hardness of the portion to be measured is calculated. Similar signal processing sections as disclosed in, for example, JP Patent Laid-open No. H09-145691 and WO 01/84135 can be applied for the signal processing section according to the present invention. In the following, an example of the principle to measure a change in a phase in a self-oscillation circuit will be explained. FIG. 13 is a graph showing an overall frequency-gain-phase characteristic curve obtained by conflating the respective frequency characteristics of a self-oscillation circuit comprising the sensor section 10 and the signal processing circuit section 21 and the phase-shifting circuit section 21. In this graph, the horizontal axis represents a frequency and the vertical axis represents a gain and a phase, respectively. The frequency-gain characteristic curve TG is an overall frequency characteristic curve obtained by conflating the frequency characteristic of the phase-shifting circuit section 21 and the frequency characteristic of the self-oscillation circuit. As shown in FIG. 13, the frequency-gain characteristic curve describes an arc-shaped curve, in which the gain is raised with the increase of the frequency in the band at the low frequency side, reaches to the maximum in the band of the resonance frequency f₀, and decreases in the band at the high frequency side. The characteristic curve θ₁₁ is a phase characteristic indicating an input/output phase difference that is a difference between the input phase and output phase of the self-oscillation circuit. In the self-oscillation circuit, an adjustment is made so that the input/output phase difference of the self-oscillation circuit comes to zero at the resonance frequency f₀ that indicates the maximum gain value TGP of the frequency-gain characteristic curve TG. That is, an adjustment is made in the phase-shifting circuit section 21 so that the conflated input/output phase difference θ₁₁, that is a phase difference between the output phase (θ₁) at the resonance frequency of the radiated oscillation from the sensor section 10 and the input phase (θ₂) being an output from the phase-shifting circuit section 21 and to be feedbacked to the sensor section 10, comes to zero (θ₁₁= θ₁+θ₂= 0) in the self-oscillation circuit. With such a manner, when a phase difference exists between the output phase and the input phase, the feedbacks are repeatedly carried out at the time of measuring the portion to be measured in the compulsory feedback loop until the conflated input/output phase difference θ₁₁ comes to zero, and an oscillation is carried out at the time that the conflated input/output phase difference θ₁₁ reaches zero. As a result, the feedback oscillation of the self-oscillation circuit can be done in more assured manner and can be promoted.

FIG. 14 is a graph showing a frequency-gain-phase characteristic curve indicating the respective frequency characteristics of the self-oscillation circuit comprising the sensor section 10 and the signal processing section 20 and the phase-shifting circuit section 21. The horizontal axis represents a frequency and the vertical axis represents a gain and a phase, respectively. As shown in FIG. 14, the frequency-gain characteristic curve 13G of the phase-shifting circuit section 21 describes an arc-shaped curve, in which the gain is raised with the increase of the frequency in the band at the low frequency side, reaches the maximum in the band of the central frequency f₂, and decreases in the band at the high frequency side. The characteristic curve θ₁₃ represents phase characteristics that indicate the input/output phase difference in the phase-shifting circuit section 21. The characteristic curve MG is a frequency-gain characteristic curve of the self-oscillation circuit excluding the phase-shifting circuit section 21. Though the central frequency f₁, frequency band and maximum gain value of the frequency-gain characteristic curve MG differ, the curve MG basically describes an arc-shaped curve that is similar to the curve of the frequency characteristic of the phase-shifting circuit section 21. As indicated with the frequency-gain characteristic curves MG and 13G shown in FIG. 14, the central frequency f₁ of the self-oscillation circuit indicated by the maximum gain value P₁ and the central frequency f₂ indicated by the maximum gain value 13GP of the phase-shifting circuit section 21 are set up to a frequency band, that is intentionally shifted, in the signal processing section. For example, the central frequency f₂ of the phase-shifting circuit section 21 is set up to a frequency that is higher than that of the central frequency f₁ of the self-oscillation circuit so that that gain increases with the increase of the hardness coefficient of the portion to be measured.

In the signal processing section set up as described above, the frequency characteristic of the reflective oscillation reflected from the portion to be measured, said reflective oscillation is received by the sensor section 10, changes in accordance with the hardness of the portion to be measured. Then, resulting from the change in the frequency characteristic, all of the frequency, gain phase and amplitude of the self-oscillation circuit change. Namely, the frequency of the self-oscillation circuit changes from the central frequency f₁ to the resonance frequency f₁₁ in accordance with the hardness of the portion to be measured. In the example shown in FIG. 14, the frequency has been increased. Further, the maximum gain value of the frequency-gain characteristic curve MG of the self-oscillation circuit changes from the maximum gain value P₁ along the frequency-gain characteristic curve 13G of the phase-shifting circuit section 21. Therefore, in the example shown in FIG. 14, the frequency-gain characteristic curve MG of the self-oscillation circuit, the maximum gain value P₁ and the gain G₁ change to ascend to MG₁, P₁₁, and to G₁₁, respectively. Since the conflated input/output phase difference θ₁₁ is adjusted to be zero in the phase-shifting circuit section 21, the frequency further changes until reaching the stable point of the feedback oscillation at which θ₁₁ comes to zero, and the gain also changes further. Consequently, the frequency-gain characteristic curve MG₁ changes to MG₂, and the resonance frequency f₁₁ changes to f₁₂. As a result, the maximum gain value P₁₁ changes to P₁₂, and the gain G₁₁ changes to G₁₂. Since the feedback loop of the self-oscillation circuit is a circuit including a resistance element and a capacity element, Δθ inevitably exists between the input phase θ₁ and the output phase θ₂, the central frequency f₁ and the gain G₁ change in series to f₁₂ and G₁₂, respectively, each of those changes equivalent to the phase difference Δθ. In the example shown in FIG. 14, these frequencies change to the ascending direction. As a result, an amount of frequency change Δf and an amount of gain change ΔG are obtained in the self-oscillation circuit. At the time point where the amount of frequency change and the amount of gain change in the self-oscillation circuit are obtained, respectively, the conflated input/output phase difference comes to zero, and the self-oscillation circuit executes feedback oscillation. Then, the said amount of frequency change Δf is taken out from the phase-shifting circuit section 21, and based on which, a hardness of the portion to be measured can be measured. The amount of frequency change Δ f that shifts due to the stiffness effect to the plus side is measured when the portion to be measured is a hard substance, whereas the amount of frequency change that shifts to the minus side due to the mass effect is measured when the portion to be measured is a soft substance. In the case of the example described above, since the phase difference of the reflective oscillation with respect to the emitted oscillation differs in accordance with the hardness of the portion to be measured, the amount of frequency change Δf and the phase difference Δθ change in accordance with the hardness, and it is possible to catch these changes by amplifying the changes. Thus, as described above, the signal processing section measures the hardness of the portion to be measured from the amount of frequency change, that is, the amount of phase change of the emitted oscillation of the oscillation section and the reflective oscillation of the oscillating wave reception section based on the prefixed correlation of the hardness and the amount of change.

Besides, instead of using the feedback circuit, it may be configured such that the input and output terminals of the sensor section 10 are connected to a DSP (Digital Signal Processor) 25, and the DSP 25 is used to process the phase difference of the emitted oscillation and the reflective oscillation by means of a software to cosmetically configure the feedback circuit and to calculate the hardness of the portion to be measured. As described above, the signal processing section 20 may be the one executing any signal processing as far as it can calculate the hardness of the portion to be measured on the basis of the phase change of the emitted oscillation and the reflective oscillation.

Note that the signal processing section may be one that detects amounts of changes of L and C at measuring the portion to be measured from the resonance frequency f₀=1/(2π√LC) of the resonance circuit on the signal processing section and calculates the hardness of the portion to be measured based on the detected amounts of changes.

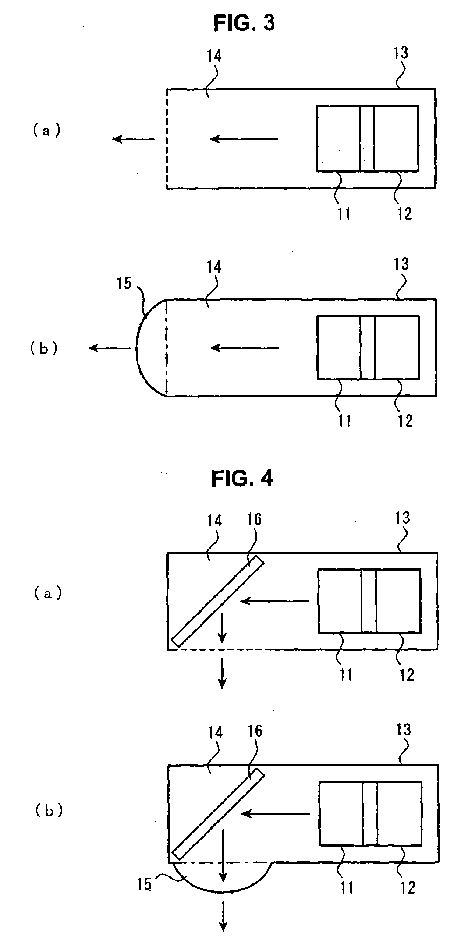

Now, the detail structure of the sensor section 10 will be specifically explained in the following. FIG. 3(a) shows only the sensor section of the catheter sensor for hardness measurement use according to the present invention. The sensor section 10 comprises an oscillation section and an oscillating wave reception section both of those which are housed in a casing 13, and the casing 13 is filled with a kind of liquid 14. The liquid 14 is specifically physiological saline solution or the like in case of a catheter sensor for in vivo uses. The oscillation generated by the oscillation section 11 is emitted to the portion to be measured through the liquid 14, and the reflective oscillation reflected from the portion to be measured is received by the oscillating wave reception section 12 through the liquid 14. In this connection, in case of the sensor shown in FIG. 3 (a), it is configured such that the emitting direction of the oscillation from the sensor section 10 is directed to the longitudinal direction of the catheter sensor and the hardness of the portion to be measured that comes to locate at the front of the catheter sensor can be measured. It is also configured such that an oscillation emission section locating at the tip portion of the casing 13 transmits the oscillation to emit it to the portion to be measured, whereas the other part of the casing 13 reflects or absorbs the oscillation. Alternatively, as shown in FIG. 13(b), an expandable balloon section 15 made from a thin film or the like may be provided to a part of the casing 13. The balloon section 15 is configured such that it expands as shown in FIG. 3(b) in response to the increase in the pressure of the liquid 14 filled in the casing 13 to thereby contact with the portion to be measured and transmits the oscillation having been transmitted via the liquid 14 directly to the portion to be measured. At operating the catheter sensor, the balloon section 15 may be made shriveled when the catheter sensor is inserted into a blood vessel, and the pressure of the liquid 14 may be raised at measuring to expand the balloon section 15 so that the balloon section 15 contacts with the portion to be measured. With the configuration as described above, an assured emission of the oscillation to the portion to be measured can be realized.

Besides, as shown in FIG. 4(a), a reflective plate 16 may be placed in order to measure a hardness of an inner wall of a blood vessel and the like existing in a direction perpendicular to the longitudinal direction of the catheter sensor. The reflective plate is placed preferably at an angle of 45° so that the oscillation emitted from the oscillation section 11 reflects in a direction perpendicular to the longitudinal direction of the catheter sensor. The oscillation emitted from the oscillation section 11 is transmitted through the liquid 14 to reach the reflective plate 16, turned at right angle to reflect there and then emitted to the portion to be measured via the oscillation emission section of the casing 13. Then, the reflective oscillation reflected from the portion to be measured reaches the reflective plate 16, turned at right angle to reflect there and then received by the oscillating wave reception section 12 following to being transmitted through the liquid 14. Note that, similarly to FIG. 3(b), it may also be configured as shown in FIG. 4(b) such that a balloon section 15 is provided to the casing 13 so that a pressure in the liquid 14 filled in the casing 13 is raised to expand the balloon section 15 to thereby directly cause the balloon section 15 in contact with the portion to be measured in an inner wall of a blood vessel locating in a direction perpendicular to the longitudinal direction of the catheter sensor for measuring the hardness.

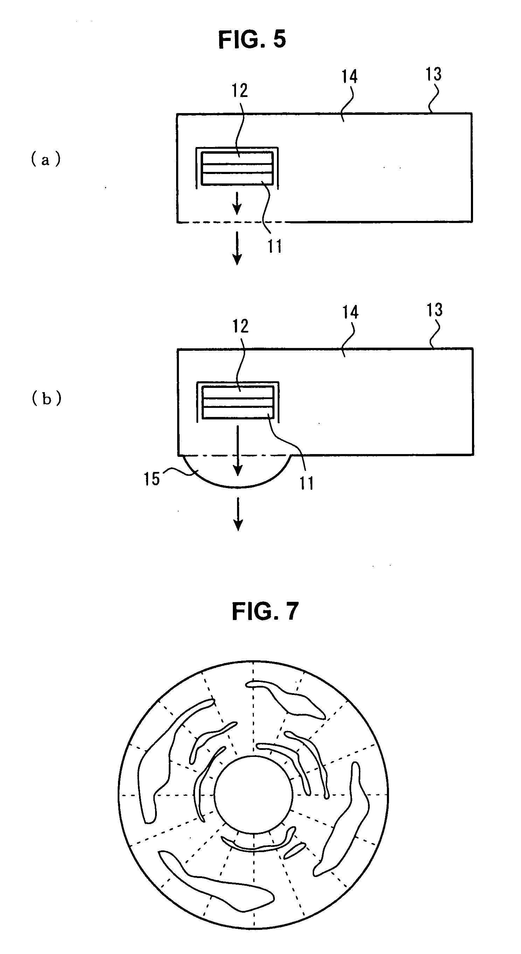

In addition, without using the reflective plate having been explained with referring to FIG. 4, it may be configured, as shown in FIG. 5(a), such that the emitting direction of the oscillation section 11 is directed to a direction perpendicular to the longitudinal direction of the catheter sensor to thereby measure the hardness of an inner wall of a blood vessel and the like. In this case as well, the balloon section 15 may be provided to the casing as shown in FIG. 5(b).

The catheter sensor for hardness measurement use configured as described above, though it can measure a hardness of a part of an inner wall of a blood vessel, cannot measure a specific position or the like, for example, what part on the circumference in an inner wall of a blood vessel is a tumor located since it has no directionality. Therefore, a catheter sensor provided with the directionality and being capable of measuring hardness of all parts on the circumference of an inner wall of a blood vessel will now be explained hereunder.

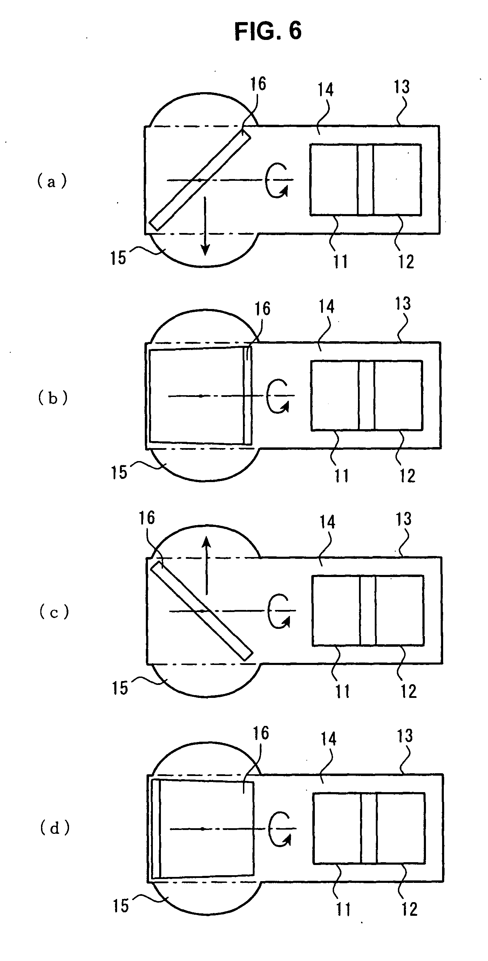

FIG. 6 shows the catheter sensor which is configured by rendering the reflective plate 16 of the catheter sensor shown in FIG. 4 rotatable about the longitudinal direction of the catheter sensor as an axis. FIG. 6 (a) shows that the reflective plate 16 being positioned so that it emits oscillation downward in the figure. When the reflective plate 16 is rotated about the longitudinal direction of the catheter sensor as an axis, it is changed to emit oscillation toward the back side in the figure as shown in FIG. 6(b). When the reflective plate is further rotated, it is changed to emit oscillation upward in the figure as shown in FIG. 6(c). Then, the reflective plate is rotated still further, it is changed to emit oscillation endways in the figure as shown in FIG. 6(d), and is returned to the state shown in FIG. 6 (a) by a further rotation. In this manner, it is made possible to measure the hardness of an inner wall of a blood vessel at overall angles from 0 through 360° around the catheter sensor by revolving the reflective plate 16 about the longitudinal direction of the catheter sensor as an axis. Since the direction of emitting oscillation and the hardness of an inner wall of a blood vessel can be measured with such a configuration, it will be made possible to display the state as to the hardness of a blood vessel three-dimensionally and in detail in combination with information on the degree of insertion of the catheter (such as the length and distance). For example, since the display can be made in red color that becomes gradually stronger with the increase in hardness of the portion to be measured or in blue color that becomes gradually stronger with the increase in softness of the portion to be measured, a determination can be made visually at once, for example, as to which part of a blood vessel does tumor exist. Contrary to visual diagnosis of an inner wall of a blood vessel using an endoscope or the like, this determination by means of the display makes a diagnosis possible that is comparable to a diagnosis performed by conducting palpation. Note that, though the casing 13 having the balloon section 15 for contacting the sensor with an inner wall of a blood vessel to measure the hardness is shown in FIG. 6, the present invention is not limited to this configuration, and the casing that has no balloon section and measures hardness with no contact with an inner wall may be used for the present invention.

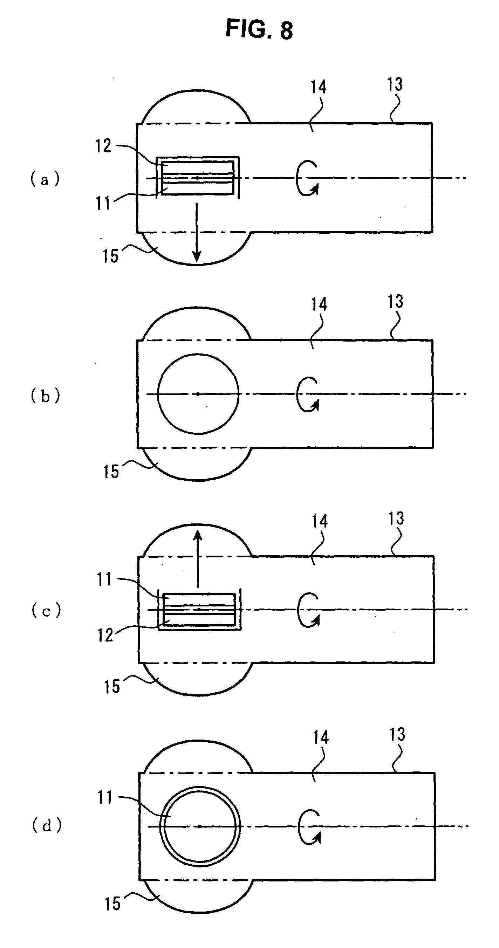

Next, a catheter sensor configured such that the emitting direction of the oscillation section 11 is directed to a direction perpendicular to the longitudinal direction of the catheter sensor as shown in FIG. 5, characterized in that the oscillation section 11 itself is rotated about the longitudinal direction of the catheter sensor as an axis to thereby capacitate the sensor to measure hardness at every points on the circumference of an inner wall of a blood vessel is shown in FIG. 8. FIG. 8(a) shows that the oscillation section 11 is positioned in a direction from which the oscillation is emitted downward in the figure. Following to rotating a wirer or the like attached to the axis of the oscillation section 11, the oscillation section 11 rotates about the longitudinal direction of the catheter sensor as an axis to emit oscillation to the backside in the figure as shown in FIG. 8(b). When the oscillation section is further rotated, the oscillation is emitted to the upward in the figure as shown in FIG. 8(c). Further, upon rotating the oscillation section still further, the oscillation is emitted to the endways in the figure as shown in FIG. 8(d), and upon rotating still further, the oscillation section returns to the state shown in FIG. 8(a). In this configuration, as well as the catheter sensor configured as shown in FIG. 6, the degree of hardness in a blood vessel can be displayed in a visible manner as shown in FIG. 7.

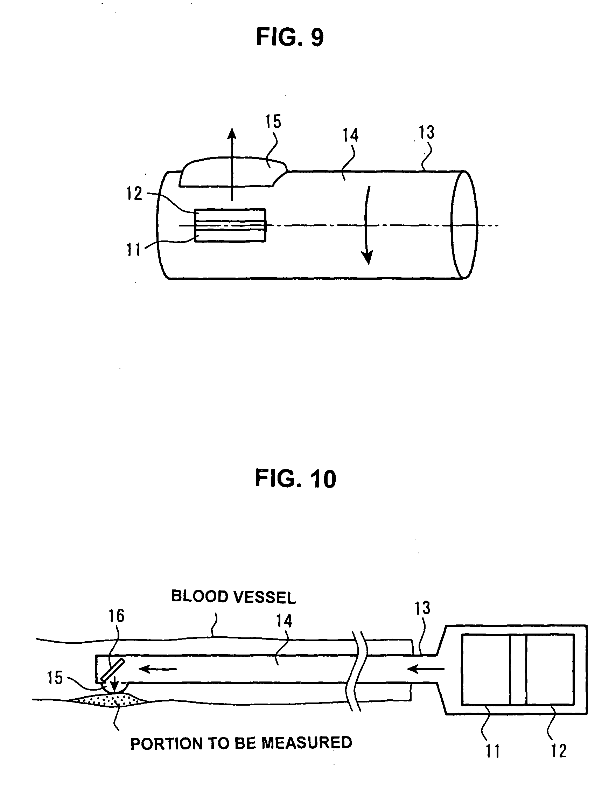

Besides, as shown in FIG. 9, it may be configured such that the casing 13 may be rotated about the longitudinal direction of the catheter sensor as an axis under such a state that the oscillation section 11 is kept fixed. Specifically, the cylinder-shaped casing 13 is formed such that the inner face thereof is formed in a reflective face so that the oscillation cannot be transmitted to the outside. Further, the oscillation section 11 is configured such that it is disposed about the longitudinal direction of the catheter sensor as an axis and emits the oscillation radially. Additionally, a balloon section 15 is provided to a section of the casing 13 so that the oscillation can be transmitted to the portion to be measured through the balloon section. Note that the balloon section 15 may be a simple thin film. With the configuration as described above, as well as the catheter sensors configured as shown in FIGS. 6 and 7, the degree of hardness in a blood vessel can be displayed visually as shown in FIG.7 in response to rotating the casing 13 about the longitudinal direction of the catheter sensor as an axis. Although an exemplary configuration in which the oscillation section 11 emits oscillation radially is shown in FIG. 9, the present invention is not limited to this example, and it may also be configured such that the oscillation section is provided to the end side part of the sensor being opposite to the part to be inserted into a blood vessel and the oscillation from the oscillation section is reflected radially in a direction perpendicular to the longitudinal direction of the catheter sensor by means of a reflective plate having a conical shape or the like.

However, in case that the catheter sensor for hardness measurement use is applied to a catheter sensor for thin blood vessels use having a diameter of 0.5 mm or less, there is a case that the sensor section cannot be installed in the catheter. Even such a case, with the catheter sensor for hardness measurement use according to the present invention, it is possible to optionally separate the positions of the part reaching the portion to be measured and a vibrator comprising the oscillation section and the oscillating wave reception section as shown in FIG. 10 since the liquid 14 is filled in the casing 13. Namely, the oscillation section and the oscillating wave reception section are configured such that they are positioned in a part of the catheter sensor, said part is located at the end side of the catheter sensor being opposite to the side of the portion to be measured, the later side is adapted to be inserted into a blood vessel, and is not inserted into a blood vessel. Even though the vibrator and the tip of the catheter are distanced, the oscillation emitted by the oscillation section is transmitted through the liquid 14 being filled in the casing 13 to reach the tip of the catheter. For example, in case of a casing 13 having a reflective plate 16 and a balloon section 15 as shown in FIG. 10, the oscillation emitted from the oscillation section 11 is transmitted through the liquid 14 to reach the reflective plate 16, refracted by the reflective plate 16 to a direction perpendicular to the longitudinal direction of the catheter sensor and reaches the balloon section 15 having been expanded due to the increase of pressure in the liquid. Then the reflective oscillation reflected from the portion to be measured is refracted again by the reflective plate 16, transmitted through the liquid 14, and then received by the oscillating wave reception section 12. In this example, the oscillation section and the oscillating wave reception section are not required to be inserted into a blood vessel or the like, and it is sufficient to insert only a catheter comprising a casing filled with the liquid. Therefore, since the catheter sensor for hardness measurement use according to the present invention can be thinned freely as far as it can fill the liquid therein for transmitting the oscillation into the casing, it can be applied even for a hyperfine catheter.

Now, the vibrator in the sensor section according to the present invention will be explained more specifically. The change in the phases of the emitted oscillation and the reflective oscillation may also be detected by using just one vibrator, installing a thin film electrode and a ground electrode to the vibrator, impressing an alternating current field to the thin film electrode, and taking frequency output out of the thin film electrode. In this case, however, it becomes difficult to take the phase difference out of the input/output waves accurately. Hence, a configuration as explained hereunder is preferably employed.

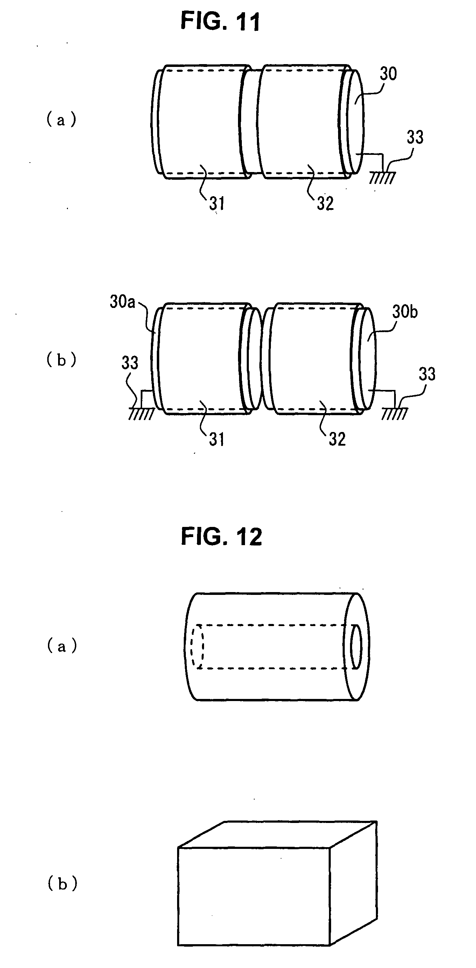

FIG. 11 shows a structure of the vibrator to be used for the sensor section in the catheter sensor for hardness measurement use according to the present invention. FIG. 11(a) is an example wherein a single vibrator is used. As shown in this figure, a thin film electrode is provided onto the vibrator by means of vacuum deposition technique or the like. Specifically, an input terminal 31 for the oscillation section, an output terminal 32 for the oscillating wave reception section and a ground terminal 33 are respectively provided to the vibrator 30 in the form of a split electrode. When an alternating current field is impressed to the input terminal 31, the vibrator oscillates to keep the oscillation at a stable frequency. Then, a frequency output of the vibrator 30 can be taken out of the output terminal 32 for the oscillating wave reception section 32. When the reflective oscillation reflecting from the portion to be measured is collided to the vibrator 30, a change appears in frequency information obtainable from the output terminal 32. The amount of the change at this time in the phases of the emitted oscillation and the reflective oscillation may be utilized for the hardness measurement of the portion to be measured.

Alternatively, two vibrators may be used as shown in FIG. 11(b). In this case, an input terminal 31 and a ground terminal 33 are provided to a vibrator 30a for the oscillation section, and an output terminal 32 and a ground terminal 33 are provided to a vibrator 30b for the oscillating wave reception section. With this configuration, it is possible to take out an amount of change in the phases of the emitted oscillation and the reflective oscillation similarly to the example described above.

Besides, various elements, such as a piezoelectric ceramic vibrator, a laminated piezoelectric ceramic vibrator, a bimorph vibrator, a crystal vibrator, a PVDF vibrator, a magnetostrictive element and an SAW, can be used for the vibrator. As to the shape of the vibrator, various shapes, such as columnar, and cylindrical and/or prismatic as shown in FIGS. 12(a) and 12(b), can be employed depending upon the shape of the casing, easiness at installing a ground terminal, etc.

Note that the catheter sensor for hardness measurement use according to the present invention is not limited to the above-described examples shown in the appended figures, and various modifications may be made naturally for the present invention within the scope that does not depart from the gist of the present invention. For example, the catheter sensor for hardness measurement use according to the present invention is naturally applicable for the hardness measurements in the intestines, other than the hardness measurements in blood vessels.

As described above, with the catheter sensor for hardness measurement according to the present invention, an excellent effect that capacitates measuring of hardness of inner walls even though the portion to be measured is a fine part such as the inside of a blood vessel can be exerted.

## Claims

1. A catheter sensor for measuring hardness of a portion to be measured in a blood vessel or the other organ comprising,
an oscillation section for generating oscillation having a prefixed frequency and emitting the generated oscillation to a portion to be measured,
an oscillating wave reception section receiving reflective oscillation reflected from the portion to be measured, wherein the oscillation is generated by the oscillation section and emitted to the portion to be measured,
a casing for containing the oscillation section and the oscillating wave reception section,
a signal processing section for calculating the hardness of the portion to be measured on the basis of a change in the phases of the emitted oscillation of the oscillation section and the reflective oscillation of the oscillating wave reception section, and
a kind of liquid to be filled in the casing,
**characterized in that** the emitted oscillation from the oscillation section is transmitted to the portion to be measured through the liquid, and the reflective oscillation from the portion to be measured is transmitted to the oscillating wave reception section through the liquid.

2. A catheter sensor for hardness measurement use according to claim 1, **characterized in that** the casing is provided with an expandable balloon section, the balloon section expands in response to an increase in the pressure of the liquid filled in the casing to contact with the portion to be measured, and the oscillation emitted from the oscillation section is transmitted through the liquid to reach the portion to be measured via the balloon section.

3. A catheter sensor for hardness measurement use according to claim 2 or 3, **characterized in that** the oscillation section and the oscillating wave reception section are positioned in a portion which locates at the end side of the catheter sensor being opposite to the side of the portion to be measured, the later side is adapted to be inserted into a blood vessel or the other organ, and is not inserted into a blood vessel or the other organ.

4. A catheter sensor for hardness measurement use according to at least one of the preceding claims, **characterized in that** the oscillation section is configured such that it emits oscillation toward the longitudinal direction of the catheter sensor, the catheter sensor has a reflection section for reflecting the oscillation from the oscillation section to a direction perpendicular to the longitudinal direction of the catheter sensor, and the reflection section is provided in the casing.

5. A catheter sensor for hardness measurement use according to claim 4, **characterized in that** the reflection section is provided so as to be rotatable about the longitudinal direction of the catheter sensor as an axis.

6. A catheter sensor for hardness measurement use according to claim 1 or 2, **characterized in that** the oscillation section is configured such that it emits oscillation to a direction perpendicular to the longitudinal direction of the catheter sensor.

7. A catheter sensor for hardness measurement use according to claim 6, **characterized in that** the oscillation section is provided so as to be rotatable about the longitudinal direction of the catheter sensor as an axis.

8. A catheter sensor for hardness measurement use according to claim 6, **characterized in that** the casing is provided so as to be rotatable about the longitudinal direction of the catheter sensor as an axis.

9. A catheter sensor for hardness measurement use according to at least one of the preceding claims, **characterized in that** the oscillation section and the oscillating wave reception section are composed of a single vibrator, the single vibrator is provided with a ground terminal, an input terminal for the oscillation section and an output terminal for the oscillating wave reception section, and the input and output terminals are composed of a split electrode.

10. A catheter sensor for hardness measurement use according to at least one of claims 1 to 8, **characterized in that** the oscillation section and the oscillating wave reception section are composed of two vibrators, one of the two vibrators is provided with an input terminal for the oscillation section and a ground terminal, and the other of the two vibrators is provided with an output terminal for the oscillating wave reception section and a ground terminal.

11. A catheter sensor for hardness measurement use according to at least one of the preceding claims, **characterized in that** the oscillation section and the oscillating wave reception section are composed of at least one of a piezoelectric ceramic vibrator, a laminated piezoelectric ceramic vibrator, a bimorph vibrator, a crystal vibrator, a PVDF vibrator, a magnetostrictive element and an SAW.

12. A catheter sensor for hardness measurement use according to at least one of the preceding claims, **characterized in that** the oscillation section and the oscillating wave reception section are respectively shaped in cylindrical, columnar or prismatic.

13. A catheter sensor for hardness measurement use according to at least one of the preceding claims, **characterized in that** the signal processing section includes a phase-shifting circuit section and an amplifier circuit section, those which are connected to between the oscillation section and the oscillating wave reception section, the oscillation section and the oscillating wave reception section together form a self-oscillation circuit, and the phase-shifting circuit section has a central frequency being different from a central frequency of the self-oscillation circuit and changes a gain with respect to a phase change at measuring the portion to be measured.

14. A catheter sensor for hardness measurement use according to at least one of claims 1 to 12, **characterized in that** the signal processing section consists of a DSP to be connected between the oscillation section and the oscillating wave reception section.
